(19) Europäisches Patentamt
European Patent Office
Office européen des brevets

(11) Veröffentlichungsnummer: **0 277 322 B1**

(12) **EUROPÄISCHE PATENTSCHRIFT**

(45) Veröffentlichungstag der Patentschrift: **07.07.93**

(51) Int. Cl.5: **C08B 37/08**, A61K 7/06, A61K 7/48

(21) Anmeldenummer: **87118464.4**

(22) Anmeldetag: **12.12.87**

Die Akte enthält technische Angaben, die nach dem Eingang der Anmeldung eingereicht wurden und die nicht in dieser Patentschrift enthalten sind.

# (54) N-Hydroxypropylisopropyläther-Derivate des Chitosans und diese enthaltende kosmetische Mittel.

(30) Priorität: **07.02.87 DE 3703760**

(43) Veröffentlichungstag der Anmeldung:
**10.08.88 Patentblatt 88/32**

(45) Bekanntmachung des Hinweises auf die Patenterteilung:
**07.07.93 Patentblatt 93/27**

(84) Benannte Vertragsstaaten:
**AT BE CH DE ES FR GB GR IT LI NL SE**

(56) Entgegenhaltungen:
**EP-A- 0 115 574**
**EP-A- 0 193 736**
**EP-A- 0 206 998**

**CHEMICAL ABSTRACTS, Band 92, Nr. 20, 19. Mai 1980, Seite 333, Zusammenfassung Nr. 169053e, Columbus, Ohio, US; & JP-A-79 132 242 (KANEBO LTD) 15-10-1979**

(73) Patentinhaber: **Wella Aktiengesellschaft**
**Berliner Allee 65**
**W-6100 Darmstadt(DE)**

(72) Erfinder: **Lang, Günther, Dr.**
**Auf der Roten Erde 10 B**
**W-6107 Reinheim 5(DE)**
Erfinder: **Maresch, Gerhard**
**Winkelschneise 6**
**W-6100 Darmstadt(DE)**
Erfinder: **Lenz, Hans-Rudi**
**Moltkestrasse 16**
**W-6100 Darmstadt(DE)**

**EP 0 277 322 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein kosmetisches Mittel zur Behandlung von Haaren oder der Haut mit einem Gehalt an neuen makromolekularen, vom Chitosan abgeleiteten Verbindungen, welche in einer geeigneten Kosmetikgrundlage zur Anwendung kommen.

Die Erfindung betrifft weiterhin neue N-Hydroxypropylisopropylether-chitosane.

Es ist bereits bekannt, in kosmetischen Mitteln, insbesondere für die Behandlung von Haaren, kationaktive Polymere, insbesondere Polymere, die quaternäre Ammoniumgruppen aufweisen, als Konditionierungsmittel einzusetzen Auf Grund einer Wechselwirkung zwischen ihren Ammoniumgruppen und den anionischen Gruppen des Haares besitzen die kationaktiven Polymere eine große Affinität zur Keratinfaser.

Es wurde festgestellt, daß der Einsatz derartiger kationaktiver Polymere in solchen kosmetischen Mitteln zahlreiche Vorteile ergibt: Die Entwirrung der Haare sowie deren Behandlung wird erleichtert, und weiterhin werden dem Haar Sprungkraft und Glanzwirkung verliehen. Durch die Affinität zum Keratin neigen diese Polymere jedoch bei wiederholter Anwendung zur Ansammlung auf den Haaren, so daß diese schwerer werden, was im Endeffekt unerwünscht ist.

Weiterhin ergeben sich bei synthetischen Polymeren Probleme wegen der physiologischen Wirkung von eventuell vorhandenen Monomerspuren, die nur schwer aus dem Polymer entfernbar sind.

Man hat bereits versucht, diese vorerwähnten Nachteile dadurch zu beheben, daß wasserlösliche Salze des Chitosans, eines durch Deacetylierung von Chitin herstellbaren Polyglucosamins, in derartigen kosmetischen Mitteln Anwendung finden. In diesem Zusammenhang wird Bezug genommen auf die eigene EP-PS 0 002 506 sowie die eigene DE-PS 26 27 419.

In gleicher Weise wie bei der Mehrzahl der kationaktiven Polymere mit quaternärer Gruppierung ergibt Chitosan ebenfalls häufig den Nachteil, daß es mit den anionaktiven oberflächenaktiven Agenzien, die üblicherweise in kosmetischen Mitteln zur Behandlung von Haaren, insbesondere in Shampoos, Anwendung finden, wenig verträglich ist. Es ist daher notwendig, das Chitosan in separaten Behandlungen, nämlich vor und/oder nach der Shampoonierung zur Einwirkung zu bringen.

Das Chitosan erweist sich weiterhin in neutralem und alkalischem Medium als praktisch unlöslich, so daß seine Anwendung beispielsweise in alkalischen Dauerwellmitteln oder Haarfärbemitteln nicht möglich ist.

Durch Einsatz von Glycidylchitosanen gemäß unserer eigenen DE-OS 32 23 423 anstelle von Chitosansalzen lassen sich die vorstehend erwähnten Nachteile zwar vermeiden, jedoch ist die Umsetzung von Chitosan mit Glycid problematisch, da Glycid in Gegenwart von Wasser rasch hydrolysiert.

Die vorstehend genannten Chitosane beziehungsweise Chitosanderivate besitzen einen weiteren Nachteil: In organischen Lösungsmitteln sind sie nicht oder nur wenig, wodurch ihre Einsatzmöglichkeiten in kosmetischen Mitteln stark eingeschränkt werden.

In der eigenen EP-OS 0 115 574 sowie der eigenen EP-OS 0 193 736 werden quaternäre Chitosanderivate beziehungsweise O-Alkyl-N-hydroxyalkyl-Chitosanderivate beschrieben, die in Wasser und organischen Lösungsmitteln löslich sein sollen und durch ein zweistufiges Verfahren beziehungsweise unter Verwendung des hydrolyseempfindlichen Glycids hergestellt werden.

Aufgabe der Erfindung ist es, aniontensidverträgliche Chitosanderivate zur Verfügung zu stellen, die sowohl für den Einsatz in wäßrigen als auch wasserfreien kosmetischen Mitteln geeignet sind.

Bei Fortführung der Untersuchungen mit Chitosan und den da von abgeleiteten Verbindungen wurde nunmehr gefunden, daß bestimmte Chitosanderivate, und zwar speziell N-Hydroxypropylisopropylether-chitosane, eine gute Aniontensidverträglichkeit aufweisen. Diese Derivate sind in Wasser und in organischen Lösungsmitteln, wie beispielsweise Ethanol und Isopropanol, löslich und ermöglichen so die Herstellung von wasserfreien, zum Beispiel alkoholischen, Lösungen.

Im Gegensatz zu synthetischen Polymeren mit endlichen Restmonomerengehalten sind diese N-Hydroxypropylisopropylether-chitosane physiologisch unbedenklich und biologisch abbaubar. Aufgrund ihrer Filmeigenschaften, ihrer Alkohollöslichkeit sowie ihrer Verdickerwirkung und Aniontensidverträglichkeit können sie nicht nur als neue, interessante Rohstoffe für Kosmetika, sondern ebenso in der Pharmazie, als Flockungs- und Verdickungsmittel in der Abwasseraufbereitung, als Appretur- und Schlichtemittel in der Textilindustrie sowie bei der Papierherstellung Anwendung finden.

Mit N-Hydroxypropylisopropylether-chitosan läßt sich somit ein kosmetisches Mittel zur Behandlung der Haare oder der Haut herstellen, das sich durch überraschend vorteilhafte Eigenschaften auszeichnet und dadurch gekennzeichnet ist, daß es in einer geeigneten Kosmetikgrundlage ein N-Hydroxypropylisopropylether-chitosan, bestehend aus

a) 4 bis 40 Molprozent Monomereinheiten der Formel (I)

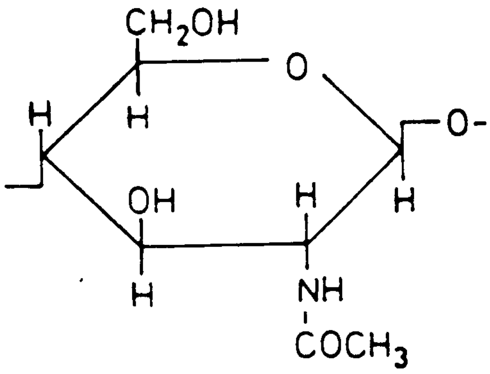

(I)

und

b) 60 bis 96 Molprozent Monomereinheiten der Formel (II)

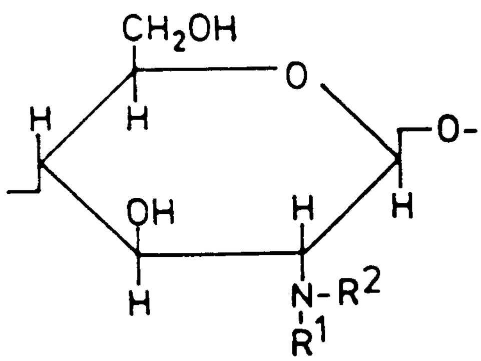

(II),

worin $R^1$ und $R^2$ gleich oder verschieden sind und Wasserstoff oder die Gruppe

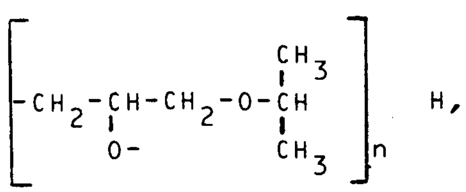

mit n gleich 1 oder 2, bedeuten, unter der Bedingung, daß bei mindestens der Hälfte der Einheiten der Formel (II) $R^1$ und $R^2$ nicht gleichzeitig Wasserstoff darstellen, enthält.

Das erfindungsgemäße N-Hydroxypropylisopropylether-chitosan enthaltende Mittel eignet sich ganz allgemein zur Behandlung der Haare und/oder der Haut. Es kann beispielsweise vorliegen als Haar- und/oder Körperwaschmittel, Tönungsshampoo, Frisiercreme, Frisierlotion, Fönlotion, Mittel zur Festigung der Frisur, Waschlotion, Haarkur, Mittel gegen Kopfschuppen, Mittel zur permanenten Haarverformung, Mittel zur Färbung oder Entfärbung von Haaren, Mittel zum Auftragen vor oder nach der Haarfärbung und als kosmetisches Mittel zur Pflege, zum Schutz oder zur Reinigung der Haut. Beispiele für ein derartiges Mittel zur Pflege und zur Reinigung der Haut sind Gesichtswässer, Rasierwässer, Feuchthaltecremes, Coldcremes, Körperlotionen, Sonnenschutzmittel oder auch Make-up-Präparate.

Der Gehalt des erfindungsgemäßen kosmetischen Mittels an N-Hydroxypropylisopropylether-chitosan liegt hierbei zweckmäßig bei 0,05 bis 10 Gewichtsprozent, vorzugsweise bei 0,05 bis 3,0 Gewichtsprozent.

Das kosmetische Mittel gemäß der vorliegenden Erfindung kann zusätzlich zu dem neuen Wirkstoff N-Hydroxypropylisopropylether-chitosan zur Herstellung einer Kosmetikgrundlage alle diejenigen Bestandteile enthalten, die in Haarund Hautbehandlungsmitteln üblicherweise eingesetzt werden, insbesondere anionische, kationische, amphotere, zwitterionische oder nichtionische oberflächenaktive Verbindungen (Tenside),

Schaumsynergisten, Stabilisatoren, Sequestriermittel, Pigmente, Verdicker, Emulgatoren, Pufferstoffe, Konservierungsmittel, Farbstoffe, Parfümöle, bekannte kosmetische Polymere wie anionische, nichtionische, kationische oder amphotere Polymere, Naturstoffe, kosmetische Öle, Fettalkohole, Wachse, Schaumstabilisatoren, Wirkstoffe gegen Kopfschuppen, Reduktionsmittel und Treibgase.

Das erfindungsgemäße kosmetische Mittel weist in bevorzugter Weise einen pH-Wert von 2 bis 12 auf und kann in Form einer wäßrigen, alkoholischen oder wäßrig-alkoholischen Zubereitung, insbesondere als Lösung, Creme, Gel, Dispersion oder Emulsion, vorliegen.

Ebenfalls ist es möglich, dieses Mittel mit Hilfe eines Zerstäubers beziehungsweise anderer geeigneter Sprühvorrichtungen oder im Gemisch mit üblichen unter Druck verflüssigten Treibmitteln als Aerosolspray (zum Beispiel Aerosolhaarspray) oder Aerosolschaum aus einem Druckbehälter zu entnehmen.

In bevorzugter Weise handelt es sich bei dem erfindungsgemäßen kosmetischen Mittel um ein Mittel zur Festigung der Frisur, wie zum Beispiel einen flüssigen Haarfestiger oder Haarspray. Dieses Mittel liegt üblicherweise als wäßrige, alkoholische oder wäßrig-alkoholische Lösung vor, die durch einen Gehalt an N-Hydroxypropylisopropylether-chitosan bestehend aus Einheiten der vorstehend genannten Formeln (I) und (II) gekennzeichnet ist. Hierbei kann das N-Hydroxypropylisopropylether-chitosan selbst als filmbildendes oder festigendes Harz eingesetzt werden; es können jedoch auch zusätzlich andere filmbildende natürliche oder synthetische kosmetische Polymere in dem erfindungsgemäßen Haarfestigungsmittel enthalten sein. Als natürliche Polymere kommen beispielsweise Schellack, Alginate, Gelatine, Pektine und Cellulosederivate in Betracht. Von den synthetischen Polymeren finden zum Beispiel Polyvinylpyrrolidon, Polyvinylacetat, Polyacrylverbindungen, wie beispielsweise Acrylsäure- oder Methacrylsäurepolymerisate, basische Polymerisate von Estern aus Acrylsäure oder Methacrylsäure mit Aminoalkoholen oder die Salze oder Quaternisierungsprodukte dieser basischen Polymerisate, Polyacrylnitril sowie Co- oder Terpolymerisate aus derartigen Verbindungen, beispielsweise Polyvinylpyrrolidon-Vinylacetat, Verwendung.

Das Mittel weist dann insbesondere einen pH-Wert zwischen 6 und 8 auf. Ein solches Mittel zur Festigung der Frisur enthält üblicherweise die filmbildenden Polymere in einer Gesamtmenge von etwa 0,05 bis 3,0 Gewichtsprozent. Enthält das Mittel neben dem hier beschriebenen N-Hydroxypropylisopropylether-chitosan aus Einheiten der vorstehend genannten Formeln (I) und (II) noch andere filmbildende Polymere, so reduziert sich der Gehalt an N-Hydroxypropylisopropylether-chitosan entsprechend.

Als Alkohole kommen insbesondere die für kosmetische Zwecke üblicherweise verwendeten niederen Alkohole mit 1 bis 4 Kohlenstoffatomen, wie zum Beispiel Ethanol und Isopropanol, in Betracht.

Wenn das Mittel zur Festigung der Frisur in Form eines Aerosolpräparates vorliegt, welches aus einem Druckbehälter versprüht wird, so enthält es in der Kosmetikgrundlage etwa 10 bis 60 Gewichtsprozent eines Treibmittels. Als Treibmittel können Chlorfluoralkane, wie zum Beispiel $CCl_3F$, $CCl_2F_2$, $C_2Cl_3F_3$, $(CCl_2F)_2$, $CHCl_2F$ und $(CClF_2)_2$, leichtflüchtige Kohlenwasserstoffe, wie zum Beispiel n-Butan und n-Propan, oder auch Dimethylether, Kohlendioxid, Distickstoffmonoxid, Stickstoff, Methylenchlorid und 1,1,1-Trichlorethan, Verwendung finden.

Das erfindungsgemäße Mittel zur Festigung der Frisur kann weiterhin die für derartige Mittel üblichen und bekannten Zusätze, wie beispielsweise Parfümöle, Bakterizide oder Fungizide, kämmbarkeitsverbessernde Substanzen und Modifiziermittel, wie zum Beispiel Siliconöl, oder Weichmacher, wie beispielsweise Isopropylmyristat, Phthalsäurediethylester und Diethylstearat, enthalten.

Das erfindungsgemäße Mittel zur Festigung der Frisur kann gegebenenfalls durch einen Gehalt an kosmetischen Farbstoffen das Haar gleichzeitig färben oder tönen. Derartige Präparate sind unter anderem als Farbfestiger oder Tönungsfestiger im Handel bekannt. Sie enthalten zusätzlich übliche für Haarfestiger bekannte, direkt auf das Haar aufziehende kosmetische Farbstoffe, wie beispielsweise aromatische Nitrofarbstoffe (zum Beispiel 1,4-Diamino-2-nitro-benzol, Pikraminsäure, 1-Hydroxy-2-amino-4-nitro-benzol und 1,4-Bis-[(2-hydroxyethyl)amino]-2-nitro-5-chlor-benzol), Azofarbstoffe (zum Beispiel C.I. 14 805-Acid Brown 4), Anthrachinonfarbstoffe (zum Beispiel C.I. 61 105-Disperse Violet 4) und Triphenylmethanfarbstoffe (zum Beispiel C.I. 42 535-Basic Violet 1), wobei die Farbstoffe dieser Klassen je nach der Art ihrer Substituenten sauren, nichtionogenen oder basischen Charakter haben können. Ihre Gesamtkonzentration in diesen Präparaten beträgt üblicherweise etwa 0,01 bis 2,0 Gewichtsprozent.

Das erfindungsgemäße Mittel zur Festigung der Frisur weist bei gleich guter Festigung des Haares gegenüber üblichen Haarfestigern eine besonders gute Kämmbarkeit und einen guten Griff des Haares im nassen Zustand sowie einen besonders angenehmen Griff des Haares im getrockneten Zustand auf.

Weiterhin kann das erfindungsgemäße Mittel ein Haarwaschmittel darstellen. Es liegt dann in Form einer wäßrigen Lösung oder Emulsion vor und enthält neben dem N-Hydroxypropylisopropylether-chitosan zumindest ein anionisches, kationisches, nichtionisches oder amphoteres Tensid.

In diesem Haarwaschmittel beträgt die Konzentration des Tensides im allgemeinen etwa 3 bis 50 Gewichtsprozent, vorzugsweise 3 bis 20 Gewichtsprozent, wobei der pH-Wert im allgemeinen zwischen 3

und 9, vorzugsweise zwischen 4 und 7, liegt.

Das erfindungsgemäße Mittel, das in Form eines Haarwaschmittels vorliegt, enthält im allgemeinen verschiedene Zusatzstoffe, insbesondere Parfüms, Konservierungsstoffe, Verdicker, Schaumstabilisatoren, Puffersubstanzen, kosmetische Harze, Pigmente und Farbstoffe.

Unter den Schaumstabilisatoren können die Fettamide und insbesondere die Mono- oder Diethanolamide von Kokosfettsäuren, Lauryl- oder Ölsäuremono- oder -diethanolamid, die zweckmäßigerweise in Mengen von 1 bis 10 und vorzugsweise von 1 bis 3 Gewichtsprozent Verwendung finden, angeführt werden.

Unter den Verdickern können insbesondere Acrylpolymere und Cellulosederivate, wie zum Beispiel Carboxymethylcellulose, Hydroxypropylmethylcellulose und Hydroxyethylcellulose, angeführt werden. Die Verdicker liegen im allgemeinen in einem Anteil von 0,1 bis 5 Gewichtprozent vor.

Unter den Tensiden oder oberflächenaktiven Agenzien, die in Kombination mit den neuen N-Hydroxypropylisopropylether-chitosanen verwendet werden, können beispielsweise die folgenden genannt werden:

a) die anionischen oberflächenaktiven Agenzien, wie beispielsweise die Alkali-, Erdalkali-, Ammonium- oder Alkanolaminsalze von Alkansulfonaten, Alkylsulfaten und Alkylethersulfaten, die $C_{12}$ bis $C_{18}$-Alkyl- und insbesondere $C_{12}$ bis $C_{14}$ Alkyl-Sulfatnatriumsalze oder -Triethanolaminsalze, die Natrium- oder Triethanolaminsalze von Lauryl- oder Tetradecylethersulfaten, das Dinatriumsalz des Sulfosuccinhalbesters von Alkanolamiden, die Seifen und die Polyethercarbonsäuren;

b) die nichtionischen oberflächenaktiven Agenzien, wie beispielsweise oxethylierte Fettalkohole mit 12 bis 18 Kohlenstoffatomen, zum Beispiel mit bis zu 40 Mol Ethylenoxid pro Mol Fettalkohol oxethylierter Lauryl-, Tetradecyl-, Cetyl-, Oleyl- und Stearylalkohol, allein oder im Gemisch; die Fettalkohole von oxethyliertem Lanolin oder oxethyliertes Lanolin; Polyglycolether von gesättigten oder ungesättigten Fettalkoholen und Alkylphenolen mit 8 bis 30 Kohlenstoffatomen im Alkylrest und 1 bis 10 Glyceryleinheiten im Molekül; Fettsäurealkanolamide sowie oxethylierte Sorbitanfettsäureester;

c) die kationischen oberflächenaktiven Agenzien, wie beispielsweise das Dilauryldimethylammoniumchlorid, die Chloride oder Bromide von Alkyldimethylbenzylammonium, die Alkyltrimethylammoniumsalze, beispielsweise Cetyltrimethylammoniumchlorid oder -bromid, die Alkyldimethylhydroxyethylammoniumchloride oder -bromide, die Dialkyldimethylammoniumchloride oder -bromide, Alkylpyridiniumsalze, beispielsweise Lauryl-oder Cetylpyridiniumchlorid, die Alkylamidethyltrimethylammoniumethersulfate, Verbindungen mit kationischem Charakter wie Aminoxide, beispielsweise Alkyldimethylaminoxide oder Alkylaminoethyldimethylaminoxide;

d) die amphoteren oder zwitterionischen oberflächenaktiven Agenzien, wie beispielsweise die Carboxylderivate von Imidazol, die N-Alkylbetaine, die N-Alkylamidobetaine, die N-Alkylsulfobetaine, die N-Alkylaminopropionate, die Alkyldimethylammoniumacetate, die $C_{12}$ bis $C_{18}$-Alkyldimethylcarboxymethylammoniumsalze sowie die Fettsäurealkylamidobetaine, beispielsweise Dimethylcarboxymethylenpropylenamido-stearat-betain.

Das erfindungsgemäße kosmetische Mittel kann auch eine Creme oder Lotion zur Verwendung als Haarkur oder Hautpflegemittel darstellen. Es liegt dann meist in Form einer Öl-in-Wasser- oder Wasser-in-Öl-Emulsion oder Suspension vor und enthält zusätzlich zu den neuen N-Hydroxypropylisopropylether-chitosanen kationische, nichtionogene, amphotere oder anionische Emulgatoren sowie als Bestandteil der Ölphase zum Beispiel Fettalkohole, Fettsäureester oder -amide, weiterhin Parfümöle, Vaseline, Wollfettalkohol oder feste beziehungsweise flüssige Paraffine.

Wenn das erfindungsgemäße Mittel ein Haartönungs- oder Haarfärbemittel darstellt, so liegt es ebenfalls bevorzugt in Form einer Creme oder Lotion vor und enthält zusätzlich übliche Haarfarbstoffe aus der Gruppe der aromatischen Nitrofarbstoffe, Azofarbstoffe, Anthrachinonfarbstoffe, Triphenylmethanfarbstoffe oder auch Oxidationsfarbstoffe, beispielsweise Resorcin und aromatische Diamine oder Aminophenole. Weiterhin kann dieses Mittel gegebenenfalls Alkalisierungsmittel, Antioxidantien sowie weitere für ein derartiges Mittel übliche kosmetische Zusätze und Hilfsstoffe enthalten.

Das erfindungsgemäße Mittel kann auch ein Dauerverformungsmittel oder Fixiermittel für Haare darstellen. Es enthält dann zusätzlich zu den genannten N-Hydroxypropylisopropylether-chitosanen ein Reduktionsmittel, wie zum Beispiel Thioglykolsäure, Thiomilchsäure und Ammoniumsulfit, oder ein Oxidationsmittel, wie zum Beispiel Hydrogenperoxid oder Natriumbromat, sowie gegebenenfalls Alkalisierungsagenzien oder Peroxidstabilisatoren, zum Beispiel Phosphorsäure, und andere kosmetische Hilfsstoffe und Zusatzstoffe, wie beispielsweise Parfümöle, Riechstoffe, Pflegestoffe und Farbstoffe.

Wie bereits erwähnt wurde, kann das erfindungsgemäße kosmetische Mittel auch zur Behandlung der Haut verwendet werden.

In der Tat erleichtert dieses kosmetische Mittel die Befeuchtung der Haut, verhindert das Austrocknen und verleiht der Haut eine hervorragende Weichheit im Griff.

Das erfindungsgemäße kosmetische Mittel liegt hierzu vorzugsweise in Form einer Creme, eines Gels, einer Emulsion oder einer wäßrigen, alkoholischen oder wäßrig-alkoholischen Lösung vor, die das N-Hydroxypropylisopropylether-chitosan in einer Konzentration von 0,1 bis 10 Gewichtsprozent und vorzugsweise von 0,2 bis 6 Gewichtsprozent enthält.

Die im allgemeinen in einer derartigen Kosmetikzubereitung enthaltenen Hilfsstoffe sind beispielsweise Duftstoffe, Farbstoffe, Konservierungsmittel, Verdickungsmittel, Sequestriermittel, Emulgiermittel, Sonnenschutzfilter und ähnliche.

Diese Zubereitung für die Hautbehandlung liegt insbesondere in Form einer Creme oder Lotion zur Pflege der Hände oder des Gesichts oder in Form einer Sonnenschutzcreme, einer gefärbten Creme, eines Abschminkmilchproduktes, eines Schaumbad- und Duschbad-Präparates oder auch in Form einer Desodorierzubereitung vor.

Diese Zubereitung wird unter Anwendung klassischer Verfahrensweisen hergestellt. Beispielsweise kann man zur Bildung einer Creme eine wäßrige Phase, die das erfindungsgemäße Chitosanderivat und gegebenenfalls andere Bestandteile oder Hilfstoffe gelöst enthält, und eine ölige Phase emulgieren. Für die ölige Phase kann man verschiedenartige Verbindungen verwenden, beispielsweise Paraffinöl, Vaselinöl, Süßmandelöl, Avocadoöl, Olivenöl, Fettsäureester, wie zum Beispiel Glycerylmonstearat, Ethylpalmitat und Isopropylpalmitat, oder Alkylmyristate, wie zum Beispiel Propylmyristat, Butylmyristat und Cetylmyristat Man kann sie auch mit Fettsäurealkoholen, beispielsweise Stearyl- oder Cetylalkohol, oder Wachsen, beispielsweise Bienenwachs oder Wollwachs versetzen.

Die N-Hydroxypropylisopropylether-chitosanderivate können in dieser Kosmetikzubereitung für die Hautpflege sowohl als Hauptwirkstoff als auch als Hilfsstoff enthalten sein.

Die in dem erfindungsgemäßen kosmetischen Mittel enthaltenen neuen N-Hydroxypropylisopropylether-chitosane leiten sich von Chitosan ab, einem Material, das durch Deacetylierung von Chitin, einem natürlich vorkommenden Acetylglucosamin, erhalten wird.

Das Chitosan ist im neutralen und alkalischen Medium unlöslich, es bildet jedoch auf Grund seiner chemischen Natur im sauren Medium mit bestimmten organischen und anorganischen Säuren lösliche Salze. Diese finden beispielsweise in der Papier- und Textilindustrie als Additive Verwendung. Weiterhin werden sie als Koagulanzien für Suspensionen, als Chelatbildner für Schwermetallionen sowie in der Medizin und in der Kosmetik benutzt (siehe in diesem Zusammenhang die Veröffentlichung von Muzarelli: "Chitin", Pergamon Press, 1977).

Es sind bereits einige wasserlösliche Chitosanderivate bekannt, beispielsweise Carboxymethylchitosan (siehe Nud'ga, Plisko und Danilov, Zhur. Obsh. Khim. 43, No. 12, Seite 2752 bis 2756 (1973); SU-PS 325 234; sowie Okimasu, Nippon Nogei Kagaku Kaishi 32, No. 5, Seite 383 bis 389 und 471 bis 473 (1958)) oder Sulfoethylchitosan (siehe Nud'ga, Plisko und Danilov, Zhur. Prikl. Khim. 47, No. 4, Seite 872 bis 875 (1974)). Diese wasserlöslichen Chitosanderivate sind jedoch entweder in ihrem ionischen Charakter verändert oder aber physiologisch bedenklich.

Hydroxyethylchitosan (Glykolchitosan) wurde von Senju und Okimasu (Nippon Nogei Kagaku Kaishi 23, Seite 432 bis 437 (1950)) bei der Glykolierung von Chitin in Gegenwart starken Alkalis durch gleichzeitige Deacetylierung erhalten.

Aufgrund niedriger Substitutionsgrade beziehungsweise Quervernetzung wasserunlösliche Hydroxyalkylderivate des Chitosans, deren stark wasserabsorbierende Eigenschaften anwendungstechnisch von Interesse sind, werden in der JP-PS 54-11 955 von 1979 erwähnt.

Schließlich beschreibt die JP-PS 57-180 602 (1982) die Synthese wasserlöslicher Chitosanderivate, die durch Reaktion von Alkylenoxiden mit Chitosan in Anwesenheit von Alkali in einem Gemisch von Wasser und einem organischen Lösungsmittel erhalten werden.

All diesen mehr oder weniger wasserlöslichen beziehungsweise wasserquellbaren Derivaten liegt die Umsetzung des Chitosans mit Alkylierungsmitteln in Gegenwart starken Alkalis zugrunde, die unter den gewählten Reaktionsbedingungen ausschließlich oder überwiegend eine O-Substitution zur Folge hat. Die zur O-Alkylierung notwendige Anwesenheit von Alkali bestimmt jedoch nicht nur den Substitutionsort, sondern bewirkt darüber hinaus, insbesondere bei höheren Temperaturen, einen Abbau der Polymerkette. Desweiteren stellen die nach der Reaktion durch Neutralisation des überschüssigen Alkalis entstehenden Salze Nebenprodukte dar, die weitere Reinigungsschritte notwendig machen.

Im Gegensatz hierzu berichtet die eigene DE-OS 32 23 423 sowie die eigene EP-OS 0 097 229 von wasserlöslichen N-substituierten Chitosanderivaten, die in bevorzugter Weise durch die Reaktion einer wäßrigen Dispersion von Chitosan mit Gycid erhalten werden. Die rasche Hydrolyse des Glycids in Gegenwart von Wasser, sein hoher Preis und die Tatsache, daß Gycid nicht großtechnisch hergestellt wird, verteuern jedoch das Verfahren zur Herstellung dieser Derivate.

Es wurde nunmehr gefunden, daß sich bei Verwendung von Gemischen aus Wasser und organischen Lösungsmitteln Chitosan auf einfache Weise mit 2,3-Epoxypropylisopropylether zu N-Hydroxypropylisopropyletherderivaten mit besonders vorteilhaften Film- und Löslichkeitseigenschaften umwandeln läßt. Im Gegensatz zu den aus unserer eigenen DE-OS 32 23 423 bekannten N-Dihydroxypropyl-chitosanen sind diese neuen N-Hydroxypropylisopropyletherderivate des Chitosans sowohl in Wasser und verdünnten Säuren als auch in Alkoholen löslich und daher auch für die Verwendung in wasserfreien, insbesondere alkoholischen, Mitteln geeignet.

In Abwesenheit basischer Katalysatoren kommt es hierbei zur Substitution der freien Aminogruppen, was durch Ermittlung des primären Amin-Stickstoffs nach van Slyke (siehe K. H. Bauer und H. Moll, "Die organische Analyse", 2. Aufl., Seite 170 bis 172, Akademische Verlagsgesellschaft Geest und Portig KG, Leipzig 1950, und H. Roth, E. v. Hulle et al. in "Analytische Methoden", Seite 674 bis 676, Georg Thieme Verlag, Stuttgart 1953) sowie durch $^{13}C$-NMR bestätigt wird.

Gegenstand der vorliegenden Erfindung sind daher sowohl wasser- als auch alkohollösliche, vom Chitosan abgeleitete N-Hydroxypropylisopropyletherverbindungen bestehend aus

a) 4 bis 40 Molprozent Monomereinheiten der Formel (I)

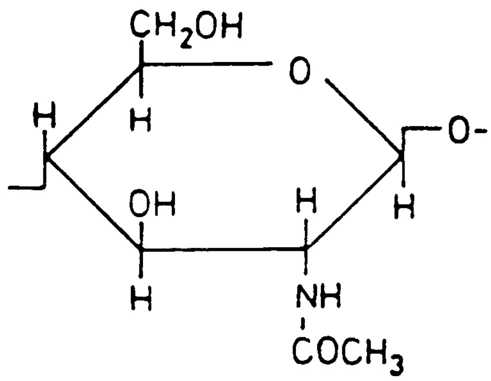

(I)

und

b) 60 bis 96 Molprozent Monomereinheiten der Formel (II)

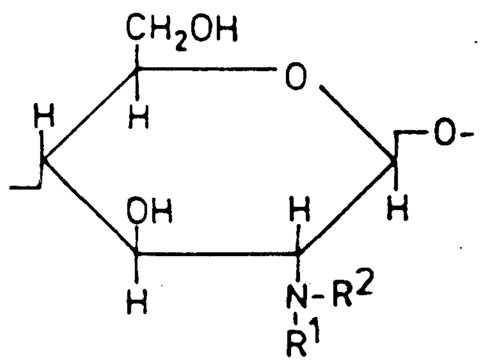

(II),

wobei $R^1$ und $R^2$ gleich oder verschieden sein können und entweder Wasserstoff oder die Gruppe

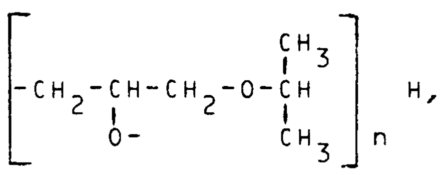

mit n gleich 1 oder 2, bedeuten, mit der Bedingung, daß bei mindestens der Hälfte der Einheiten der Formel (II) $R^1$ und $R^2$ nicht gleichzeitig Wasserstoff darstellen, oder deren wasserlösliche Salze.

Die neuen Chitosanderivate werden in Analogie zur ersten Stufe des in der eigenen DE-OS 35 04 095 beschriebenen zweistufigen Verfahrens zur Herstellung von O-Alkyl-N-Hydroxypropyl-chitosanen erhalten, indem man eine Aufschlämmung von Chitosan (zu 60 bis 96 Prozent deacetyliertes Chitin) oder seiner Salze bei Temperaturen zwischen 20 und 120 Grad Celsius, vorzugsweise zwischen 40 und 100 Grad Celsius, unter Druck im Autoklaven, in einem geeigneten Verhältnis mit 2,3-Epoxypropylisopropylether über einen Zeitraum von 3 bis 72 Stunden, vorzugsweise 6 bis 48 Stunden, umsetzt.

In bevorzugter Weise führt man die Reaktion in einem Gemisch bestehend aus Wasser und einem organischen Lösungsmittel in neutralem Medium aus. Bei Verwendung von Chitosansalzen oder von Chitosan in Anwesenheit saurer Katalysatoren, wie zum Beispiel Salzsäure, kann die Reaktion in einer Dispersion oder Lösung bestehend aus Wasser und einem organischen Lösungsmittel oder Wasser und überschüssigem 2,3-Epoxypropylisopropylether erfolgen. Das molare Verhältnis von Chitosan zu Epoxid wählt man zwischen 1 : 3 bis 1 : 15.

Nach beendeter Reaktion entfernt man das überschüssige Alkylierungsmittel, trennt eventuell vorhandene unlösliche Anteile aus den Lösungen des Chitosanderivates durch Filtration ab, neutralisiert gegebenenfalls, engt am Rotationsverdampfer ein und fällt die Chitosanderivate unmittelbar oder nach der Dialyse in Aceton aus.

Die erfindungsgemäßen N-Hydroxypropylisopropylether-chitosane können durch Neutralisierung der Aminogruppen mit anorganischen oder organischen Säuren in die entsprechenden Salze überführt werden. Gemäß der vorliegenden Erfindung sind jedoch nur solche Salze verwendbar, die in Wasser löslich sind. Geeignete Salze sind zum Beispiel solche, die mit Salzsäure, Glykolsäure, Milchsäure, Ameisensäure, Zitronensäure oder Essigsäure gebildet werden.

Die nachfolgenden Beispiele sollen den Gegenstand der Erfindung näher erläutern, ohne diesen hierauf zu beschränken.

## Herstellungsbeispiele

### Beispiel 1

50 g (0,31 mol) niedermolekulares, gemahlenes Chitosan mit einer Grenzviskositätszahl (Eta) von 160 ml/g und einem Deacetylierungsgrad von 90 Prozent werden in 400 ml Isopropanol / Wasser (1 : 1) dispergiert und im Autoklaven unter Rühren mit 144 g (156,6 ml = 1,24 mol) 2,3-Epoxypropylisopropylether versetzt. Nach 12-stündiger Reaktionszeit bei 100 Grad Celsius wird das Umsetzungsprodukt mit Hilfe eines Rotationsverdampfers zur Trockne eingeeingt und sodann bei 50 Grad Celsius im Vakuumtrockenschrank getrocknet. Anschließend wird der Rückstand in 1000 ml Ethanol gelöst. Nach Druckfiltration zur Entfernung nicht umgesetzter Anteile engt man das Filtrat im Vakuum auf 300 ml ein und fällt schließlich in der 8 bis 10-fachen Menge Aceton unter Zuhilfenahme eines Rührers aus.

Der Niederschlag wird auf einer Glassinternutsche gesammelt, gründlich mit Aceton gewaschen und bei 50 Grad Celsius im Vakuum getrocknet.

Es werden 48 g N-Hydroxypropylisopropylether-chitosan erhalten.

| Kenndaten: | |
|---|---|
| Grenzviskositätszahl (Eta) | = 60 ml/g |
| Substitutionsgrad Hydroxypropylisopropylether | = 0,9 |
| Pendelhärte | = 174 Sekunden |
| Wasserdampfaufnahme | = 4,8 Prozent |

### Beispiel 2

20 g (0,12 mol) eines hochmolekularen Chitosans mit einer Grenzviskositätszahl (Eta) von 1600 ml/g und einem Deacetylierungsgrad von 76 Prozent werden in einem Druckgefäß in 600 ml Ethylenglykoldimethylether/wasser (8 : 2) dispergiert, mit 111,5 g (121,2 ml = 0,96 mol) 2,3-Epoxypropylisopropylether versetzt und unter Rühren bei 80 Grad Celsius 20 Stunden lang zur Reaktion gebracht. Die Aufarbeitung erfolgt wie in Beispiel 1 beschrieben.

Die Ausbeute an N-Hydroxypropylisopropylether-chitosan beträgt 26,2 g.

| | |
|---|---|
| Grenzviskositätszahl (Eta)<br>Substitutionsgrad Hydroxypropylisopropylether<br>Pendelhärte<br>Wasserdampfaufnahme | = 194 ml/g<br>= 1,3<br>= 170 Sekunden<br>= 5,2 Prozent |

**Beispiel 3**

50 g (0,31 mol) niedermolekulares, gemahlenes Chitosan mit einer Grenzviskositätszahl (Eta) von 160 ml/g und einem Deacetylierungsgrad von 90 Prozent werden unter Zugabe von 43,8 g (0,3 mol) 25-prozentiger Salzsäure in 200 ml Isopropanol/wasser (1 : 1) dispergiert und in einem Autoklaven mit 209,1 g (227,3 ml = 1,8 mol) 2,3-Epoxypropylisopropylether 24 Stunden lang bei 90 Grad Celsius umgesetzt.

Die Aufarbeitung des Umsetzungsproduktes erfolgt nach der Neutralisation und Dialyse wie in Beispiel 1 beschrieben.

Es werden 44 g N-Hydroxypropylisopropylether-chitosan erhalten.

| Kenndaten: | |
|---|---|
| Grenzviskositätszahl (Eta)<br>Substitutionsgrad Hydroxypropylisopropylether<br>Pendelhärte<br>Wasserdampfaufnahme | = 35 ml/g<br>= 1,7<br>= 161 Sekunden<br>= 7,3 Prozent |

Der Substitutionsgrad für die Hydroxypropylisopropyletherreste wurde mit Hilfe der $^1$H-NMR-Spektren ermittelt.

Die Messung der Grenzviskositätszahlen erfolgte in einer wäßrigen Lösung von 0,2 mol/l Essigsäure und 0,1 mol/l Natriumacetat (Chitosan) beziehungsweise in einer wäßrigen Lösung von 0,2 mol/l Essigsäure und 0,1 mol/l Natriumchlorid (N-Hydroxypropylisopropylether-chitosan) bei 25 Grad Celsius unter Verwendung eines DIN-Ubbelohde-Kapillarviskosimeters.

Die Pendelhärte wurde nach König (W. König, "Härtemessungen mit dem Pendelhärteprüfer", Farbe und Lack 65, Seite 435 bis 443 (1959); DIN 53 157) bestimmt.

Die Wasserdampfaufnahme wurde bei 70 Prozent relativer Luftfeuchte gegenüber 30 Prozent relativer Luftfeuchte ermittelt.

**Beispiele für kosmetische Mittel**

**Beispiel 4** Haarspray (treibmittelfrei)

| | |
|---|---|
| 2,5 g | N-Hydroxypropylisopropylether-chitosan nach Beispiel 3 (Eta = 35 ml/g, Substitutionsgrad = 1,7) |
| 61,5 g | Ethanol |
| 35,5 g | Isopropanol |
| 0,5 g | Parfümöl |
| 100,0 g | |

**Beispiel 5** Haarfestiger

| | |
|---|---|
| 0,6 g | N-Hydroxypropylisopropylether-chitosan nach Beispiel 1 (Eta = 60 ml/g, Substitutionsgrad = 0,9) |
| 25,0 g | Isopropanol |
| 0,4 g | Ameisensäure (10-prozentige, wäßrige Lösung) |
| 0,2 g | Parfümöl |
| 73,8 g | Wasser |
| 100,0 g | |

20 ml dieser Lösung werden auf dem gewaschenen, handtuchtrockenen Haar verteilt. Anschließend werden die Haare in üblicher Weise zur Frisur eingelegt und getrocknet. Bei guter Festigungswirkung zeigt das Haar, im Vergleich zu einem Haarfestiger auf der Basis von Chitosan/Ameisensäure, einen angenehmeren und weicheren Griff.

**Beispiel 6** Sprühfönlotion

| | |
|---|---|
| 0,5 g | N-Hydroxypropylisopropylether-chitosan nach Beispiel 3 (Eta = 35 ml/g, Substitutionsgrad = 1,7) |
| 58,0 g | Isopropanol |
| 0,4 g | Phthalsäurediethylester |
| 0,4 g | Parfümöl |
| 0,1 g | Cetyltrimethylammoniumchlorid |
| 40,6 g | Wasser |
| 100,0 g | |

Abfüllverhältnis: 91 Prozent Wirkstoff
9 Prozent Propan/Butan (Druck: 0,27 MPa bei 20 Grad Celsius)

Die Fönlotion wird auf das gewaschene, handtuchtrockene Haar aufgesprüht. Anschließend wird das Haar in üblicher Weise gefönt und geformt. Das Haar zeigt bei guter Konditionierung gegenüber einer Fönlotion mit synthetischen Polymeren einen wesentlich angenehmeren und weicheren Griff.

**Beispiel 7** Tönungsfestiger

| | |
|---|---|
| 0,60 g | N-Hydroxypropylisopropylether-chitosan nach Beispiel 1 (Eta = 60 ml/g, Substitutionsgrad = 0,9) |
| 45,00 g | Ethanol |
| 0,15 g | 1,4-Bis-[(2-hydroxyethyl)-amino]-2-nitro-5-chlor-benzol |
| 54,25 g | Wasser |
| 100,00 g | |

20 ml dieser Lösung werden auf die gewaschenen, handtuchtrockenen Haare gegeben, sodann wird das Haar eingelegt und getrocknet. Die Haare sind rot-violett gefärbt und gefestigt.

**Beispiel 8** Anionisches Haarwaschmittel

| | | |
|---|---|---|
| 1,00 | g | N-Hydroxypropylisopropylether-chitosan nach Beispiel 2 (Eta = 194 ml/g, Substitutionsgrad = 1,3) |
| 40,00 | g | Laurylalkoholdiglykolethersulfat-Natriumsalz (28-prozentige, wäßrige Lösung) |
| 4,00 | g | Natriumchlorid |
| 0,10 | g | Formaldehyd (25-prozentige, wäßrige Lösung) |
| 0,05 | g | Farbstoff |
| 54,85 | g | Wasser |
| 100,00 | g | |

Es wird ein klares Shampoo erhalten. Das damit gewaschene Haar ist hinsichtlich Griff, Glanz und Kämmbarkeit ausgezeichnet konditioniert.

**Beispiel 9** Amphoteres, tönendes Haarwaschmittel

| | | |
|---|---|---|
| 2,00 | g | N-Hydroxypropylisopropylether-chitosan nach Beispiel 1 (Eta = 60 ml/g, Substitutionsgrad = 0,9) |
| 40,00 | g | Dimethyl-carboxymethylen-propylenamido-stearat-betain (35-prozentige, wäßrige Lösung) |
| 5,06 | g | Ameisensäure (10-prozentige, wäßrige Lösung) |
| 3,50 | g | Kokosfettsäure (einprozentige, wäßrige Lösung) |
| 1,00 | g | Pikraminsäure (einprozentige, wäßrige Lösung) |
| 48,44 | g | Wasser, vollentsalzt |
| 100,00 | g | |

Mit 15 bis 20 g des obigen Haarwaschmittels wird das Haar einshampooniert. Nach einer Einwirkungszeit von 5 bis 10 Minuten spült man mit Wasser aus. Das Haar ist gelb-orange getönt und ausgezeichnet konditioniert.

**Beispiel 10** Kationisches Haarkurmittel

| | |
|---|---|
| 0,30 g | N-Hydroxypropylisopropylether-chitosan nach Beispiel 2 (Eta = 194 ml/g, Substitutionsgrad = 1,3) |
| 4,00 g | Cetylstearylalkohol |
| 2,50 g | Kokos(pentaethoxy)methylammoniumchlorid |
| 1,48 g | Milchsäure (10-prozentige, wäßrige Lösung) |
| 1,00 g | Sorbitanmonopalmitat, mit 20 Mol Ethylenoxid oxethyliert |
| 90,72 g | Wasser, vollentsalzt |
| 100,00 g | |

35 g des Haarkurmittels nach Beispiel 10 werden im gewaschenen Haar verteilt und nach einer Einwirkungszeit von 3 bis 5 Minuten mit Wasser wieder ausgespült. Als Ergebnis werden ausgezeichneter Griff, Glanz sowie Kämmbarkeit des Haares erhalten.

**Beispiel 11** Haarkurmittel, gelförmig

| | |
|---|---|
| 2,1 g | N-Hydroxypropylisopropylether-chitosan nach Beispiel 2 (Eta = 194 ml/g, Substitutionsgrad = 1,3) |
| 0,6 g | Hydroxypropylmethylcellulose |
| 0,5 g | Laurylpyridiniumchlorid |
| 96,8 g | Wasser, vollentsalzt |
| 100,0 g | (auf pH 5,0 mit zehnprozentiger Ameisensäure eingestellt) |

Die Anwendung des Gels erfolgt wie in Beispiel 9 beschrieben wurde. Als Ergebnis werden Griff, Glanz und Kämmbarkeit des Haares wesentlich verbessert.

**Beispiel 12** Haartönungsmittel

| | | |
|---|---|---|
| 0,30 | g | N-Hydroxypropylisopropylether-chitosan nach Beispiel 1 (Eta = 60 ml/g, Substitutionsgrad = 0,9) |
| 12,00 | g | Cetylstearylalkohol |
| 6,00 | g | Laurylalkoholdiglykolethersulfat-Natriumsalz (28-prozentige, wäßrige Lösung) |
| 0,85 | g | 1,4-Diamino-2-nitro-benzol |
| 0,50 | g | Parfümöl |
| 0,50 | g | 1-Hydroxy-2-amino-4-nitro-benzol |
| 0,24 | g | Natriumhydroxid |
| 0,10 | g | 4-Hydroxy-benzoesäureethylester |
| 79,51 | g | Wasser |
| 100,00 | g | |

30 bis 40 g des obigen Haartönungsmittels werden in dem gewaschenen Haar verteilt und nach einer Einwirkungszeit von etwa 20 Minuten ausgespült. Das Haar ist rötlich gefärbt und weist eine gute Kämmbarkeit und einen angenehmen Griff auf.

**Beispiel 13** Oxidationshaarfärbemittel

| | | |
|---|---|---|
| 0,50 | g | N-Hydroxypropylisopropylether-chitosan nach Beispiel 2 (Eta = 194 ml/g, Substitutionsgrad = 1,3) |
| 15,00 | g | Cetylalkohol |
| 3,50 | g | Laurylalkoholdiglykolethersulfat-Natriumsalz (28-prozentige, wäßrige Lösung) |
| 3,00 | g | Ammoniak (25-prozentige, wäßrige Lösung) |
| 0,30 | g | 1,4-Diamino-benzol |
| 0,30 | g | Natriumsulfit |
| 0,25 | g | Resorcin |
| 0,08 | g | 3,5-Diamino-2,6-dimethoxy-pyridin-dihydrochlorid |
| 77,07 | g | Wasser |
| 100,00 | g | |

50 g dieses Haarfärbemittels werden mit 50 ml 6-prozentiger Hydrogenperoxidlösung gemischt und auf weißes Haar aufgetragen. Nach 30 Minuten wird das Haar mit Wasser ausgespült und getrocknet. Das Haar hat eine natürlich wirkende matt-blonde Färbung sowie einen natürlichen, angenehmen Griff erhalten.

**Beispiel 14** Dauerwellmittel

| | |
|---|---|
| 0,5 g | N-Hydroxypropylisopropylether-chitosan nach Beispiel 3 (Eta = 35 ml/g, Substitutionsgrad = 1,7) |
| 10,0 g | Thioglykolsäure |
| 8,0 g | Ammoniak (25-prozentige, wäßrige Lösung) |
| 6,1 g | Ammoniumhydrogencarbonat |
| 75,4 g | Wasser |
| 100,0 g | |

Zur Anwendung trägt man dieses Dauerwellmittel auf das gewickelte, handtuchtrockene Haar gleichmäßig auf und läßt es etwa 20 Minuten lang einwirken. Danach wird das Haar mit Wasser ausgespült und in bekannter Weise oxidativ behandelt. Es wird ein gutes Wellergebnis erhalten, und die Haare fühlen sich natürlich und weich an.

**Beispiel 15** Hautcreme

| | |
|---|---|
| 0,30 g | N-Hydroxypropylisopropylether-chitosan nach Beispiel 1 (Eta = 60 ml/g, Substitutionsgrad = 0,9) |
| 3,00 g | Stearylalkohol |
| 1,00 g | Wollwachs (Adeps Lanae) |
| 1,00 g | Vaseline |
| 1,00 g | Natriumacetylstearylsulfat |
| 0,76 g | Milchsäure (10-prozentige, wäßrige Lösung) |
| 92,94 g | Wasser, vollentsalzt |
| 100,00 g | |

Alle Prozentangaben in der vorliegenden Anmeldung stellen, soweit nicht anders angegeben, Gewichtsprozente dar.

**Patentansprüche**

**1.** Kosmetisches Mittel zur Behandlung der Haare und der Haut, welches einen pH-Wert von 2 bis 12 aufweist, dadurch gekennzeichnet, daß es in einer geeigneten Kosmetikgrundlage ein N-Hydroxypropylisopropylether-chitosan, bestehend aus

a) 4 bis 40 Molprozent Monomereinheiten der Formel (I)

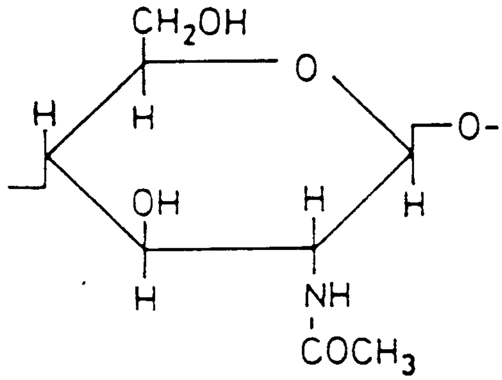

(I)

und

b) 60 bis 96 Molprozent Monomereinheiten der Formel (II)

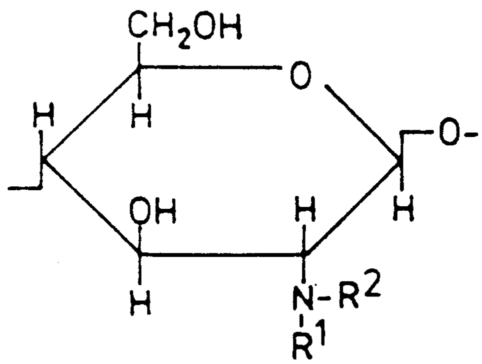

(II)

worin $R^1$ und $R^2$ gleich oder verschieden sind und Wasserstoff oder die Gruppe

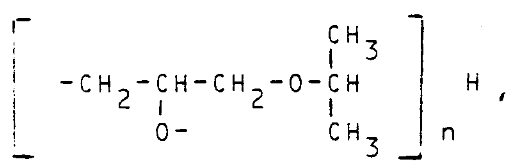

mit n gleich 1 oder 2, bedeuten, unter der Bedingung, daß bei mindestens der Hälfte der Einheiten der Formel (II) $R^1$ und $R^2$ nicht gleichzeitig Wasserstoff darstellen, enthält.

**2.** Mittel nach Anspruch 1, dadurch gekennzeichnet, daß es das N-Hydroxypropylisopropylether-chitosan in einer Menge von 0,05 bis 10,0 Gewichtsprozent enthält.

**3.** Mittel nach Anspruch 1 und 2, dadurch gekennzeichnet, daß es in Form einer wäßrigen, alkoholischen oder wäßrig-alkoholischen Zubereitung, insbesondere als Lösung, Creme, Gel, Dispersion oder Emulsion, vorliegt.

**4.** Mittel nach Anspruch 1 bis 3, dadurch gekennzeichnet, daß es zusätzlich ein bekanntes filmbildendes synthetisches oder natürliches kosmetisches Polymer enthält.

**5.** Mittel nach Anspruch 1 bis 4, dadurch gekennzeichnet, daß es zusätzlich mindestens einen kosmetischen Farbstoff in einer Konzentration von 0,01 bis 2,0 Gewichtsprozent enthält und in Form eines Farbfestigers oder Tönungsfestigers vorliegt.

6. Mittel nach Anspruch 1 bis 5, dadurch gekennzeichnet, daß es als Kosmetikgrundlage eine wäßrige, alkoholische oder wäßrig-alkoholische Zubereitung enthält, die mit einem unter Druck verflüssigten Treibmittel vermischt ist, in einem Druckbehälter abgefüllt ist und in Form eines Aerosolsprays oder -schaums vorliegt.

7. Mittel nach Anspruch 1 bis 3, dadurch gekennzeichnet, daß es zusätzlich mindestens ein kationisches, nichtionisches, amphoteres oder anionisches Tensid enthält und in Form eines Haarwaschmittels vorliegt.

8. Mittel nach Anspruch 7, dadurch gekennzeichnet, daß es das Tensid in einer Konzentration von 3 bis 50 Gewichtsprozent enthält und einen pH-Wert zwischen 3 und 9 aufweist.

9. Makromolekulare, vom Chitosan abgeleitete N-Hydroxypropylisopropyletherverbindung bestehend aus
a) 4 bis 40 Molprozent Monomereinheiten der Formel (I)

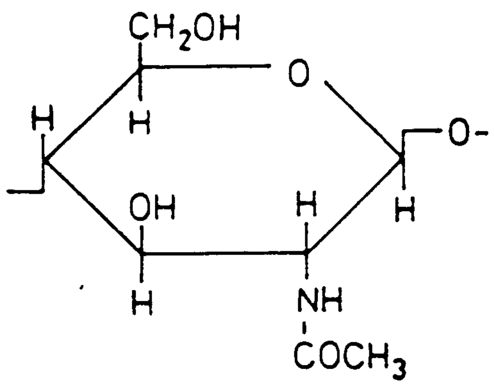

(I)

und
b) 60 bis 96 Molprozent Monomereinheiten der Formel (II)

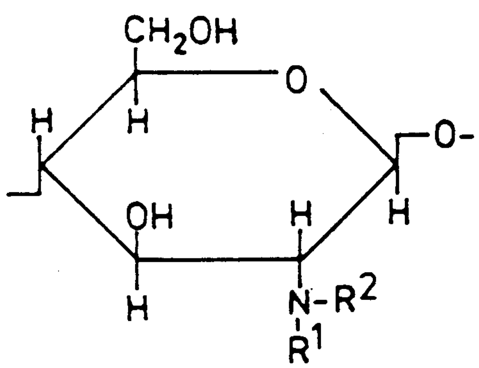

(II),

wobei $R^1$ und $R^2$ gleich oder verschieden sind und entweder Wasserstoff oder die Gruppe

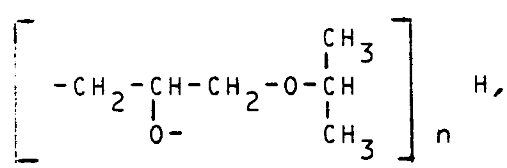

mit n gleich 1 oder 2, bedeuten, mit der Bedingung, daß bei mindestens der Hälfte der Einheiten der Formel (II) $R^1$ und $R^2$ nicht gleichzeitig Wasserstoff darstellen, oder deren wasserlösliches Satz.

## Claims

1. Cosmetic composition for the treatment of the hair and skin having a pH-value of 2 to 12, characterised in that it contains, in a suitable cosmetic base, an N-hydroxypropylisopropylether-chitosan, consisting of
   (a) 4 to 40 molar percent of monomer units of formula (I)

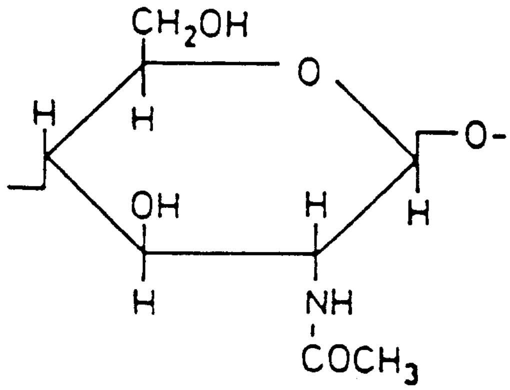

(I)

and
   (b) 60 to 96 molar percent of monomer units of formula (II)

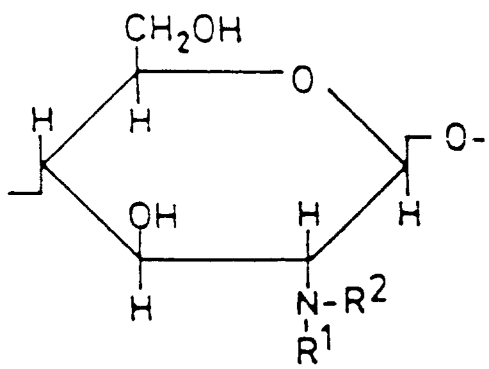

(II)

in which $R^1$ and $R^2$ are the same or different and represent hydrogen or the group

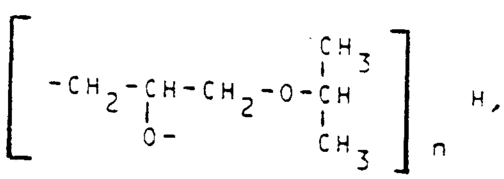

with n being equal to 1 or 2, provided that in at least half of the units of formula (II), $R^1$ and $R^2$ do not both represent hydrogen.

2. Composition according to Claim 1, characterised in that it contains the N-hydroxypropylisopropylether-chitosan in an amount of from 0.05 to 10.0 weight percent.

3. Medium according to Claim 1 and 2, characterised in that it is in the form of an aqueous, alcoholic, or aqueous-alcoholic preparation, especially a solution, cream, gel, dispersion, or emulsion.

4. Composition according to Claim 1 to 3, characterised in that it contains additionally a known synthetic or natural film-forming cosmetic polymer.

5. Composition according to Claim 1 to 4, characterised in that it additionally contains a cosmetic dye in a concentration of from 0.01 to 2.0 weight percent and in the form of a dye fixer or tint fixer.

6. Composition according to Claim 1 to 5, characterised in that it contains as cosmetic base an aqueous, alcoholic, or aqueous-alcoholic preparation, which is mixed with a propellant that is liquefied under pressure, is charged into a pressure container and is in the form of an aerosol spray or aerosol foam.

7. Composition according to Claim 1 to 3, characterised in that it additionally contains at least one cationic, non-ionic, amphoteric or anionic surface active agent and is in the form of a hair shampoo.

8. Composition according to Claim 7, characterised in that it contains the surface active agent in a concentration of from 3 to 50 weight percent and has a pH value between 3 and 9.

9. Macromolecular chitosan-derived N-hydroxypropylisopropylether compound consisting of
(a) 4 to 40 molar per cent of monomer units of the formula (I)

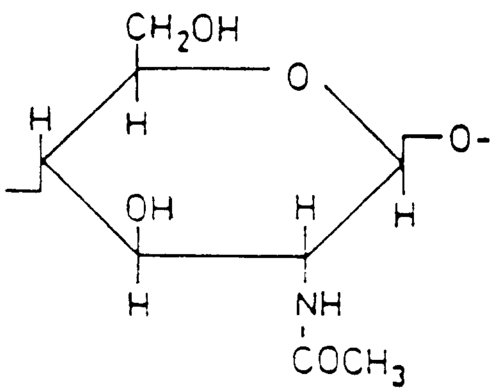

(I)

and
(b) 60 to 96 molar percent of monomer units of formula (II)

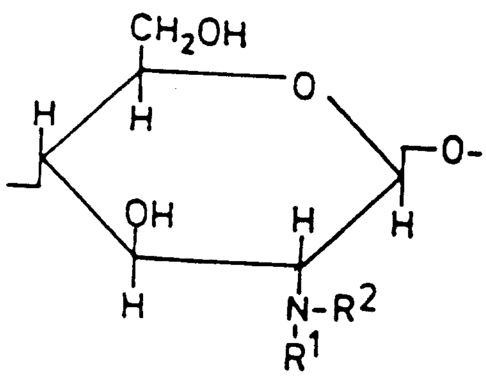

(II),

wherein $R^1$ and $R^2$ are the same or different and mean either hydrogen or the group

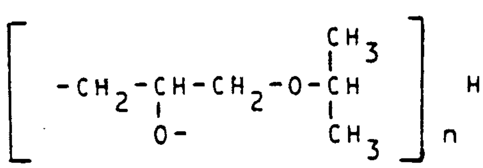

,

with n being equal to 1 or 2, provided that in at least half of the units of formula (II) $R^1$ and $R^2$ are not both hydrogen, or water soluble salts thereof.

## Revendications

1. Produit cosmétique pour traiter les cheveux et la peau d'un pH de 2 à 12, caractérisé en ce qu'il contient, dans une base cosmétique appropriée, un N-hydroxypropylisopropyl-éther-chitosane, constitué par
   (a) un pourcentage molaire compris entre 4 et 40 de la formule (I) et

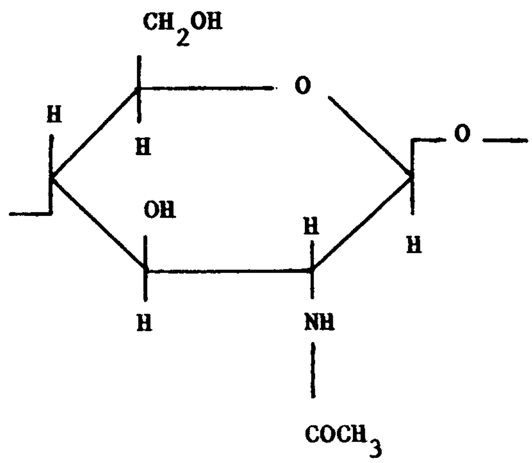

( I )

   (b) un pourcentage molaire compris entre 60 et 96 de la formule (II)

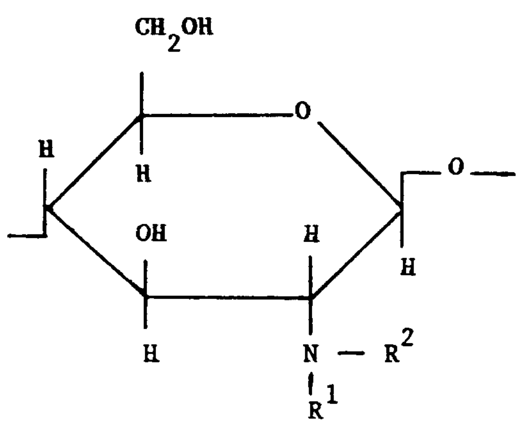

( II ),

   dans laquelle $R^1$ et $R^2$ sont identiques ou différents, et sont de l'hydrogène ou le groupe

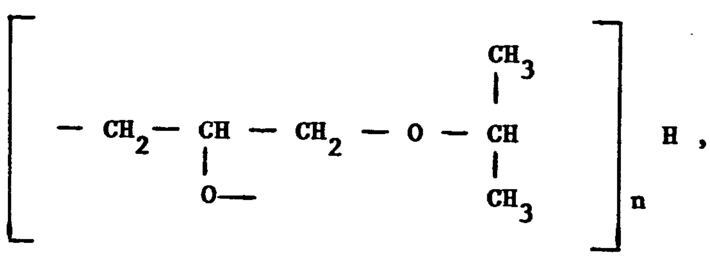

   dans laquel n est égal à 1 ou 2, à la condition que, pour au moins la moitié des unités de la formule (II) $R^1$ et $R^2$ ne représentent pas en même temps de l'hydrogène.

2. Produit selon la revendication 1, caractérisé en ce qu'il contient le N-hydroxypropylisopropyléther-chitosane en une quantité comprise entre 0,05 et 10,0 pourcent en poids.

3. Produit selon la revendication 1 et 2, caractérisé en ce qu'il se présente sous la forme d'une préparation aqueuse, alcoolique ou aqueuse-alcoolique, notamment en tant que solution, crème, gel, dispersion ou émulsion.

4. Produit selon l'une des revendications 1 à 3, caractérisé en ce qu'il contient en plus un polymère cosmétique connu formant un film, synthétique ou naturel.

5. Produit selon l'une des revendications 1 à 4, caractérisé en ce qu'il contient en plus au moins un colorant cosmétique en une concentration comprise entre 0,01 et 2,0 pourcent en poids et se présente sous la forme d'un fixateur de couleur ou d'un fixateur de tonalité.

6. Produit selon l'une des revendications 1 à 5, caractérisé en ce qu'il contient en tant que base cosmétique une préparation aqueuse, alcoolique ou aqueuse-alcoolique, qui est mélangée à un produit moussant liquéfié sous pression, est versée dans un récipient sous pression et se présente sous la forme d'un spray aérosol ou d'une mousse aérosol.

7. Produit selon l'une des revendications 1 à 3, caractérisé en ce qu'il contient en plus au moins un agent tensio-actif cationique, non-ionique, amphotère ou anionique et se présente sous la forme d'un produit de lavage pour les cheveux.

8. Produit selon la revendication 7, caractérisé en ce qu'il contient l'agent tensio-actif en une concentration comprise entre 3 et 50 pourcent en poids et qu'il a un pH compris entre 3 et 9.

9. Composé macromoléculaire de N-hydroxypropylisopropyléther dérivé du chitosane, constitué par
(a) un pourcentage molaire compris entre 4 et 40 de la formule (I)

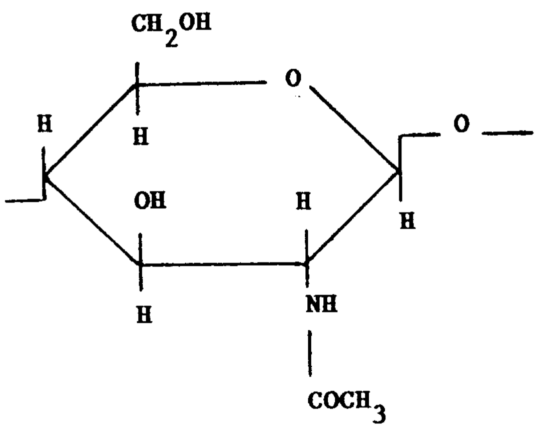

( I )

et
(b) un pourcentage molaire compris entre 60 et 96 d'unités monomères de la formule (II)

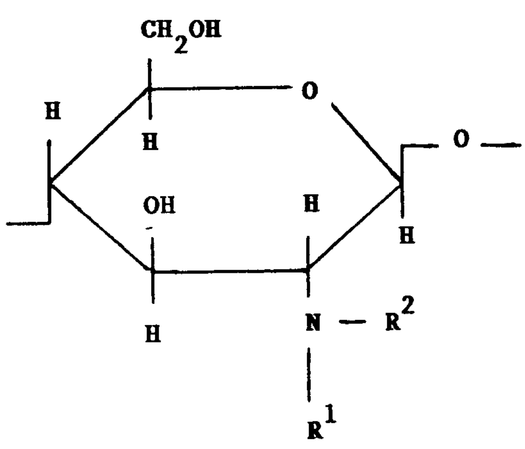

( II ),

dans laquelle $R^1$ et $R^2$ sont identiques ou différents et sont soit de l'hydrogène soit le groupe

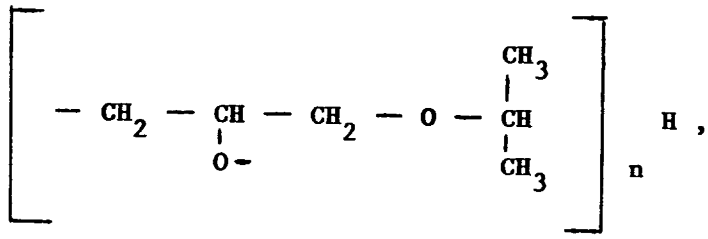

dans lequel n est égal à 1 ou 2, à la condition que pour au moins la moitié des unités de la formule (II), $R^1$ et $R^2$ ne représentent pas en même temps de l'hydrogène, ou un de leurs sels solubles dans l'eau.